# EUROPEAN PATENT APPLICATION

(11) **EP 4 403 192 A1**
(43) Date of publication of application: **24.07.2024**
(21) Application number: 21957301.1
(22) Date of filing: 19.11.2021
(51) Int. Cl.: A61K 49/00, A61K 47/02

(54) **APPLICATION OF IRON OXIDE NANOPARTICLES IN PREPARATION OF PARATHYROID AND/OR LYMPH NODE CONTRAST AGENT**

(30) Priority: 18.09.2021 CN 202111101501
(71) Applicant: Nonsuch Biotechnology, Inc., Hangzhou, Zhejiang 310000 (CN)
(72) Inventor: ZHENG, Weihui, Hangzhou, Zhejiang 310000 (CN); GUO, Peng, Hangzhou, Zhejiang 310000 (CN); QIN, Jiangjiang, Hangzhou, Zhejiang 310000 (CN); CHENG, Xiangdong, Hangzhou, Zhejiang 310000 (CN)
(74) Representative: Canzler & Bergmeier Patentanwälte Partnerschaft mbB
(86) International application number: PCT/CN2021/131855
(87) International publication number: WO 2023/040037

(57) **Abstract**

An application of iron oxide nanoparticles (IONPs) in the preparation of a parathyroid and/or lymph node contrast agent, wherein the particle size of the IONPs is 1-1000 nm, the polydispersity index is <0.3, the zeta-potential is -50 to 50 mV, and the peak value of an ultraviolet-visible absorption spectrum is 100-500 nm. The IONPs may accurately negatively visualize parathyroid glands and trace lymph nodes.

## Description

### FIELD OF DISCLOSURE

The present disclosure relates to the technical field of surgical navigation imaging technology, and relates to a negative contrast agent for parathyroid glands, preparation and use thereof, especially to an iron oxide nanoparticle, preparation and use thereof in a negative contrast agent for parathyroid glands.

### DESCRIPTION OF RELATED ARTS

In 2021, the GLOBOCAN reported that the incidence of thyroid gland cancers ranked ninth among all cancers in the world and fifth among female cancers. According to the statistics of imaging data from 25 countries around the world reported by the sub-journal of the *Lancet,* the incidence of all major thyroid gland cancer subtypes has increased significantly in China. At the same time, low-risk papillary thyroid gland cancers have increased rapidly around the world, including China.

Surgery is the main treatment for thyroid gland cancers. With the rapidly increasing incidence of the thyroid gland cancers, related surgeries have increased significantly. Although the parathyroid glands are small, the short-term and long-term postoperative complications caused by injury of parathyroid glands are extremely severe. It is essential for patients if the parathyroid glands can be better imaged during surgical procedures. In thyroid gland surgeries, due to the anatomical characteristics of the parathyroid gland itself and the close relationship between the parathyroid gland and the thyroid gland, the cases of damage to parathyroid gland functions increase with the increased number of thyroid gland surgeries. Because parathyroid glands are derived from different pharyngeal pouches during embryonic development, there are many anatomical variations, especially in the lower parathyroid gland, which can be ectopic to the mandibular angle, the anterior mediastinum, or even the pericardium, or can also be embedded in the thymus or thyroid gland. The in-situ preservation of parathyroid gland remains highly challenging. Therefore, how to protect the parathyroid gland better during surgery is one of the clinical problems to be solved urgently. To date, this remains a clinically important, unmet need.

Currently, limitations of clinical intraoperative imaging of parathyroid glands are shown as follows.

The current intraoperative imaging of the parathyroid glands mainly includes both positive imaging and negative imaging. The positive contrast agents include methylene blue, aminolaevulinic acid, indocyanine green, etc. However, the positive contrast agents lack the specificity for parathyroid glands, generally resulting in a low staining rate and poor recognition of normal parathyroid glands, and have side effects such as arrhythmia, neurotoxicity, iodine allergy, etc.

The negative contrast agents are mainly carbon nanomaterials, which are a kind of black carbon nanoparticles (CNPs). Such contrast agents function to negatively image the parathyroid glands by staining the normal thyroid gland to black. It has been used clinically in China. However, its use in non-tumor surgeries is restricted, and its use in secondary surgeries is even more difficult. In addition, carbon nanomaterials have severe potential safety issues that can cause harm to human bodies: carbon nanomaterials cannot be degraded normally in human bodies, which can damage DNA during cell replication, and may induce cancers. Thus, the safety of carbon nanomaterials in human has remained controversial. During common thyroid gland microcarcinoma surgeries, the applied carbon nanomaterials penetrate into lateral cervical lymph nodes through the lymphatic vessels in the thyroid gland area, which are not dissected, therefore, the carbon nanomaterials will remain in the lymph nodes in this area for a long time. Moreover, a long-lasting stains in the skin caused by injection of carbon nanomaterials may occur in endoscopic thyroid surgeries.

In recent years, there have been many studies investigating the toxicity of carbon nanomaterials. In 2020, Cheng Wenting reported the genome instability of peripheral blood lymphocytes caused by long-term exposure to CNPs in her master's thesis. In 2019, Dr. Zhang Rui from Shandong University detailedly discussed the toxicity of CNPs at molecular, cellular, and animal levels in his dissertation "Ultrafine Carbon Black Inducing Oxidative Stress and Genotoxicity at Molecular, Cellular, and Animal Levels*".* It was found that CNPs can induce apoptosis of primary liver cells in mice, enter the primary liver cells in mice, inhibit cell viability, and induce a toxic effect. CNPs can also result in a reduction in photosensitivity of the visual cells of zebrafishes, cause toxicity to the glial cells in the nervous system of zebrafishes, and lead to disorders in the movement behavior of zebrafishes. In 2018, Zhao Dandan illustrated the effect of CNPs combined with cold stimulation on the respiratory system and body energy metabolism of mice in her master's thesis. In 2017, Zhou Lixiao showed in her master's thesis that CNPs have a toxic effect on human bronchial epithelial cells. In addition, it has also been reported that carbon nanomaterials such as carbon nanotubes and graphene have reproductive toxicity, cause vascular endothelial dysfunction and epithelial cell damage, affect human pleural mesothelial cells, and so on. Therefore, it becomes an urgent clinical need to develop a nontoxic, harmless, biologically degradable contrast agent for parathyroid glands.

### SUMMARY OF THE PRESENT INVENTION

In view of the above-mentioned shortcomings of the prior art, an object of the present disclosure is to provide iron oxide nanoparticles (IONPs) and preparation and use thereof in the negative imaging of parathyroid glands. The IONPs can help better preserve the parathyroid glands during surgery to maintain their function. It has been proven that the iron oxide nanoparticles can be degraded by the lysosomes of normal human cells and digested into iron ions, which can combine with ferritin and then enter the normal iron metabolism, so that IONPs can be completely absorbed and degraded by human bodies, demonstrating its safety. For example, Ferumoxytol IONPs have been approved by the FDA for the clinical treatment of iron deficiency anemia (IDA) in adults.

To achieve the above object and other related objects, the present disclosure provides iron oxide nanoparticles (IONPs) with physicochemical properties of: a nanoparticle structure with a particle size of 1 nm to 1,000 nm, a polydispersity index of less than 0.3, a zeta-potential of -50 mV to 50 mV, and a peak value of ultraviolet-visible absorption spectrum of 100 nm to 500 nm.

In the iron oxide nanoparticles (IONPs) of the present disclosure, the particle size is in a range of 1 nm to 1,000 nm; e.g., 1-5 nm, 1-10 nm, 1-50 nm, 1-100 nm, 1-200 nm, 5-50 nm, 5-100 nm, 5-200 nm, 10-50 nm, 10-100 nm, 10-200 nm, 10-20 nm, 20-30 nm, 30-40 nm, 40-50 nm, 50-60 nm, 60-70 nm, 70-80 nm, 80-90 nm, 90- 100 nm, 100-110 nm, 110-120 nm, 120-130 nm, 130-140 nm, 140-150 nm, 150-200 nm, 200-250 nm, 250-300 nm, 350-400 nm, 450-500 nm, 550-600 nm, 650-700 nm, 750-800 nm, 850-900 nm, 900-950 nm, or 950-1000 nm. Preferably, the particle size is in a range of 1-200 nm. More preferably, the particle size is in a range of 1-100 nm. Still more preferably, the particle size is in a range of 1-30 nm. Still more preferably, the particle size is in a range of 10-30 nm. Still more preferably, the particle size is in a range of 10-20 nm. Still more preferably, the particle size is 10 nm.

In the iron oxide nanoparticles (IONPs) of the present disclosure, the zeta-potential is in a range of -50 mV to 50 mV; e.g., -50 mV to 40 mV, -40 mV to -30 mV, -30 mV to -20 mV, -20 mV to -10 mV, -20 mV to 20 mV, -10 mV to 0 mV, 0 mV to 10 mV, 10 mV to 20 mV, 20 mV to 30 mV, 30 mV to 40 mV, or 40 mV to 50 mV; preferably, -35 mV to 20 mV; more preferably, -35 mV to 0 mV; more preferably, -30.57 mV.

In the iron oxide nanoparticles (IONPs) of the present disclosure, the peak value of the ultraviolet-visible absorption spectrum is in a range of 100 nm to 500 nm; e.g., 100-120 nm, 120-140 nm, 140-160 nm, 160-180 nm, 180-200 nm, 200-220 nm, 200-300 nm, 220-240 nm, 240-260 nm, 260-280 nm, 280-300 nm, 300-320 nm, 320-340 nm, 340-360 nm, 360-380 nm, 380-400 nm, 400-420 nm, 420-440 nm, 440-460 nm, 460-480 nm, or 480-500 nm. Preferably, the peak value of the ultraviolet-visible absorption spectrum is in a range of 180-300 nm. More preferably, the peak value of the ultraviolet-visible absorption spectrum is in a range of 200-260 nm. Still more preferably, the peak value of the ultraviolet-visible absorption spectrum is in a range of 200-220 nm.

Further, to achieve the excellent performance of the iron oxide nanoparticles (IONPs) of the present disclosure in the imaging, the IONPs can also have a specific polydispersity index. Preferably, the polydispersity index is less than 0.3; eg: 0.01, 0.02, 0.03, 0.04, 0.05, 0.06, 0.07, 0.08, 0.09, 0.10, 0.12, 0.14, 0.16, 0.18, 0.20, 0.22, 0.24, 0.26, or 0.28. More preferably, the polydispersity index is in a range of 0.01-0.10. More preferably, the polydispersity index is in a range of 0.01-0.05. More preferably, the polydispersity index is in a range of 0.01-0.03. Still more preferably, the polydispersity index is 0.01, 0.02, or 0.03. Still more preferably, the polydispersity index is 0.03.

The present disclosure further provides a use of the iron oxide nanoparticles (IONPs) in the preparation of a negative contrast agent for parathyroid glands.

The present disclosure further provides a use of the iron oxide nanoparticles (IONPs) in the preparation of a positive contrast agent for lymph nodes.

The present disclosure further provides a contrast agent, where the negative contrast agent includes the iron oxide nanoparticles (IONPs) described above. The contrast agent can serve as a positive contrast agent for thyroid gland, a negative contrast agent for parathyroid glands, or a positive contrast agent for lymph nodes.

The IONPs of the present disclosure can be preliminarily screened for their diagnostic efficacy by *in vivo* or *in vitro* tests, and other methods are also obvious to those skilled in the art.

The present disclosure can utilize any iron oxide nanoparticles (IONPs) that have the above properties. Iron oxide nanoparticles (IONPs) can be prepared by other methods, which include a thermal decomposition method with a general procedure including heating iron oxide powder and oleic acid in octadecene at high temperature to form IONPs, and then mixing the IONPs in organic solvent with aqueous solvent under reflux to enable the IONPs into the aqueous solvent to obtain the IONPs.

To practice the use disclosed herein, the IONPs can be administered alone or in a form of a composition as prepared.

The composition can be prepared as a contrast agent or a diagnostic tracer for diagnostic purposes. Alternatively, the composition can be prepared as a pharmaceutical composition for therapeutic purposes. In addition to the above-mentioned IONPs, the composition can further include one or more pharmaceutically acceptable carriers or media. The carriers and media need to be compatible with the IONPs. The acceptable carriers or media can include, e.g., sterile water or normal saline, stabilizers, excipients, antioxidants (ascorbic acid, etc.), buffers (phosphoric acid, citric acid, other organic acids, etc.), preservatives, surface active agents (PEG, Tween, etc.), chelating agents (EDTA, etc.), adhesives, polymers, etc. Moreover, it can also include other low molecular weight peptides; proteins such as serum albumin, gelatin, or immunoglobulin; amino acids such as glycine, glutamine, asparagine, arginine, and lysine; sugars or carbohydrates such as polysaccharides and monosaccharides; and sugar alcohols such as mannitol or sorbitol. When an aqueous solution for injection is prepared, normal saline, an isotonic solution containing glucose or other auxiliary drugs (such as D-sorbitol, D-mannose, D-mannitol, and sodium chloride), as well as an appropriate solubilizer (such as alcohol (ethanol, etc.), polyol (propylene glycol, PEG, etc.) and a nonionic surfactant (Tween 80, HCO-50)) can be used.

The present disclosure further provides a use of the composition in diagnosis of thyroid gland and/or lymph node-associated diseases or disorders, tissue imaging of thyroid gland or lymph nodes, or intraoperative navigation.

In one embodiment, the diagnostic tracer composition can also be prepared into a detection kit.

In one embodiment, the IONPs are prepared along with a drug in a form of pharmaceutical composition for tracking the targeting and therapeutic effect of the drug.

In the pharmaceutical composition of the present disclosure, the amount of IONPs is usually a safe and effective amount, which can be adjustable by those skilled in the art. For example, the administration dose of the IONPs usually depends on the patient's weight, application type, as well as status and severity of the disease. For example, the administration dose of IONPs can usually be 0.1-1 mg/kg/time.

The compositions provided by the present disclosure (i.e., containing contrast agents or diagnostic tracers, or pharmaceutical compositions, etc.) can be suitable for any administration mode, which can be oral or parenteral administration, e.g., pulmonary, nasal, rectal, and/or intravenous injection, more specifically, intradermal, subcutaneous, intramuscular, intra-articular, intraperitoneal, pulmonary, oral, sublingual, nasal, transdermal, vaginal, oral or parenteral administration. Injection administration includes intravenous injection, intramuscular injection, subcutaneous injection, transdermal administration, etc.

As used herein, the dosage form of the composition is selected from the group consisting of injections, sterile powders for injection, tablets, pills, capsules, lozenges, spiritus, powders, granules, syrups, solutions, tinctures, aerosols, powder spray, or suppository. Those skilled in the art can select an appropriate dosage form according to the administration mode. For example, dosage forms suitable for oral administration can include, but are not limited to pills, tablets, chewables, capsules, granules, solutions, drops, syrups, aerosols, or powder sprays, etc.; as another example, dosage forms suitable for parenteral administration can include, but are not limited to solutions, suspensions, reconstitutable dry preparations or sprays, etc.; as another example, dosage forms suitable for rectal administration can usually be suppositories; and as another example, dosage forms suitable for injection administration can be injections, sterile powders for injection, etc.

Further, when the contrast agent or diagnostic tracer is used in diagnosis, the meaning of "diagnosis" includes, but is not limited to, diagnosing or detecting the presence of thyroid gland diseases, or performing an intraoperative real-time surgical navigation or observing the drug targeting by imaging, and so on.

The IONPs of the present disclosure can be used in various diagnostic imaging methods for thyroid glands, including ultrasound, magnetic resonance, and X-ray imaging. They can be used in ultrasonic diagnostic imaging and magnetic resonance imaging.

To the contrast agent or diagnostic tracer of the present disclosure can be added various additives, such as emulsifiers, coating agents, plasticizers, fillers, cryoprotectants, and/or antioxidants, e.g., to improve the stability, dispersibility, aggregation tendency, and biological properties thereof, or to improve the flexibility and/or polarity of the membrane.

The present disclosure further provides a method for negatively imaging parathyroid glands, including: administering the IONPs of the present disclosure to an individual.

The present disclosure further provides a method for imaging lymph nodes, including: administering the IONPs of the present disclosure to an individual.

The principle for negative imaging of the parathyroid glands in the present disclosure includes: parathyroid glands are small and have large anatomical variations, are located on the back side of the thyroid gland, and are closely related to the thyroid gland. The structure of parathyroid glands is high condensed with cells. In comparison, the structure of thyroid glands is relatively loose with lumens and interstitial spaces. After performing an injection in the thyroid gland area, the IONPs can readily diffuse into the thyroid gland but cannot enter the parathyroid gland due to the tissue structure difference between the parathyroid gland and thyroid gland, which negatively images the parathyroid gland immediately adjacent to the thyroid gland. In combination with the function of positively imaging the lymph nodes, the lymph nodes at the inferior thyroid gland can be imaged by black staining of IONPs, so that the parathyroid gland at the inferior thyroid gland can be negatively imaged.

The principle for imaging of the lymph node in the present disclosure includes: after the local injection of IONPs (in the lower lip), the IONPs are transported to the submandibular lymph nodes via the anterior cervical lymphatic vessels due to lymphatic drainage. The submandibular lymph nodes in rats are relatively superficial, and thus the IONP-stained lymph nodes can be observed by incising the skin.

The present disclosure further provides a method for diagnosing thyroid gland and/or lymph nodes using the IONPs as described above, including administering the IONPs or composition as described herein as a tissue contrast agent, and obtaining an image of the mammal after the administration of the IONPs or compositions. In some embodiments, the method further includes evaluating a signal level after administering the diagnostic tracer or contrast agent (including the IONPs or composition as described herein) with a control signal level.

The present disclosure further provides a method for preventing and/or treating diseases or conditions associated with thyroid gland and/or lymph nodes, including administering an effective amount of the IONPs or composition described herein to a mammal, and the method can further include acquiring an image of the mammal after the administration of the IONPs or composition. The method can also be *in vitro* or non-therapeutic.

The present disclosure further provides a method for screening drugs that are effective in treating thyroid gland and/or lymph nodes. By constructing the IONPs and the drug molecules into the same carrier such as microspheres, evaluating the positive imaging effect on the thyroid gland, the negative imaging effect on the parathyroid gland, and/or the positive imaging effect on the lymph node of the IONPs to determine the targeting of the drug molecules on the thyroid gland and/or lymph node diseases to screen the drugs effective in the treatment of thyroid gland and/or lymph nodes. The method of the present disclosure has excellent operability, good accuracy, etc.

Unless otherwise defined, all technical and scientific terms used herein have the same meaning as familiar to one skilled in the art. In addition, any method or material similar or equivalent to those described can be used in the method of the present disclosure. The embodiments and materials described herein are only used for illustrative purposes.

The terms "include," "comprise", or similar terms herein should be understood to be inclusive, rather than exclusive or exhaustive, that is, they should be understood as "including, but are not limited to".

As used herein, "iron oxide nanoparticles," "iron oxide nanometric particles" and "nano-iron oxide" have the same meaning.

As used herein, "carbon nanoparticles (CNPs)," "carbon nanometric particles" and "nano carbon" have the same meaning.

The terms "treatment" and "prevention" herein should be understood in their broadest sense. The term "treatment" does not necessarily imply that the mammal is treated until fully recovered. Similarly, the "prevention" does not necessarily mean that the subject will not eventually be infected with the disease or disorder. Thus, the treatment and prevention include alleviating symptoms of a specific disorder or preventing or reducing the risk of developing a specific disorder. The term "prevention" can be understood as reducing the severity of the attack of a specific disorder. The treatment can also be understood as reducing the severity of an existing disorder or the frequency of an acute attack.

In the present disclosure, the subject or individual to be therapeutically or prophylactically treated is preferably a mammal, e.g., but not limited to humans, primates, livestock (such as sheep, cattle, horses, donkeys, pigs), pets (such as dogs, cats), laboratory test animals (such as mice, rabbits, rats, guinea pigs, hamsters) or captured wild animals (such as foxes, deer). The subject is preferably a primate. The subject is most preferably human.

Other features, objects, and advantages can be easily known with reference to the specification, accompanying drawings, and claims.

The iron oxide nanoparticles (IONPs) of the present disclosure and preparation and use thereof have the following beneficial effects:
(1) The iron oxide nanoparticles (IONPs) of the present disclosure can be used to accurately negatively image parathyroid glands and trace lymph nodes. Compared with positive control carbon nanoparticles, the IONPs of the present disclosure are degradable, difficult to leak out, would not contaminate the surgical field, and have clearer images, when being used as a contrast agent.
(2) The iron oxide nanoparticles of the present disclosure are degradable and safe, and can be metabolized by the human body, without affecting normal cells and secondary operations; which can be made into reagents for intravenous injection.
(3) The iron oxide nanoparticles (IONPs) of the present disclosure can reduce postoperative complications such as numbness of hands and feet, general convulsion, epilepsy, neurasthenia, diaphragmatic spasm, and permanent hypoparathyroid gland glandism caused by operation.
(4) The iron oxide nanoparticles (IONPs) of the present disclosure help to solve problems that are urgently required to be addressed but not yet solved, and have broad applications and high clinic translation values.
(5) The method for preparing the iron oxide nanoparticles (IONPs) of the present disclosure is simple to operate, low cost, and has uniform product quality, good repeatability, and is suitable for scalable production.

### BRIEF DESCRIPTION OF THE DRAWINGS

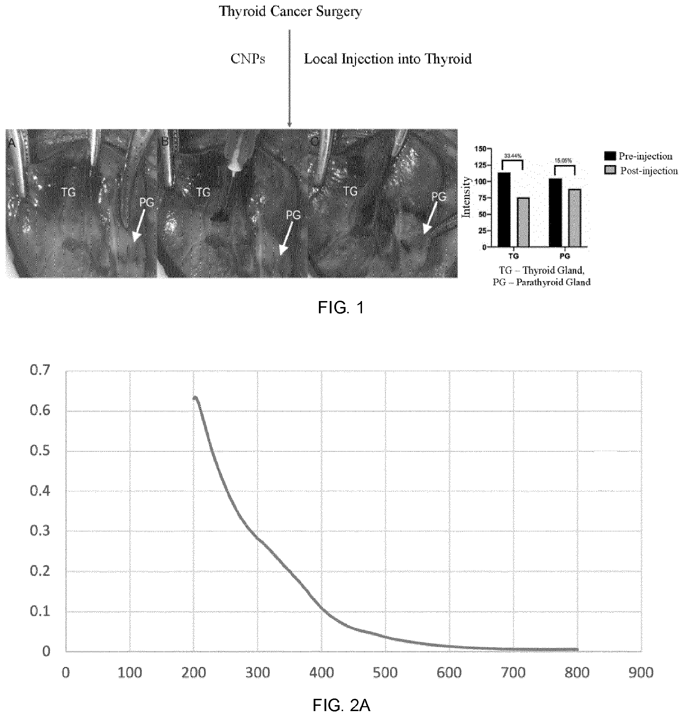

FIG. 1 is a scene diagram of a clinical thyroid gland cancer surgery utilizing carbon nanoparticles, in which the histogram shows a grayscale comparison before and after the injection of nano carbon in a thyroid gland cancer surgery for humans.

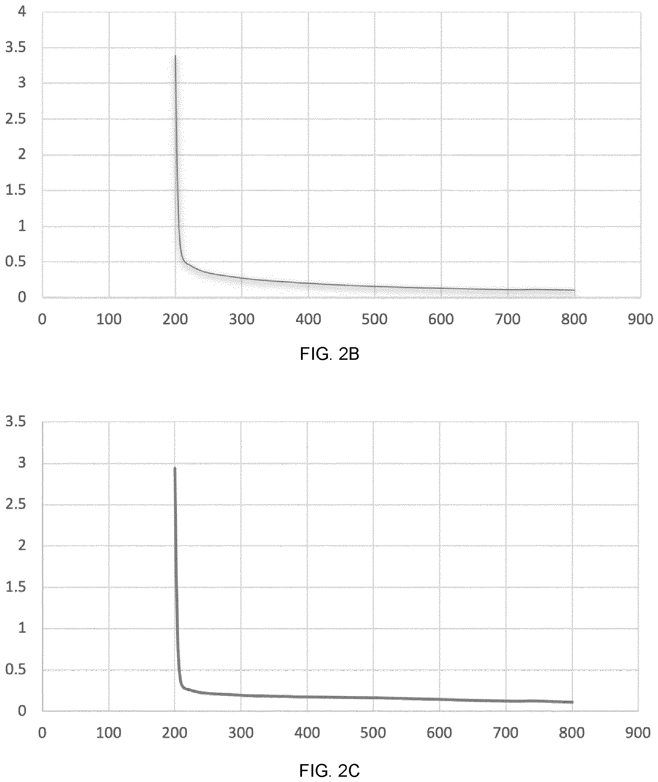

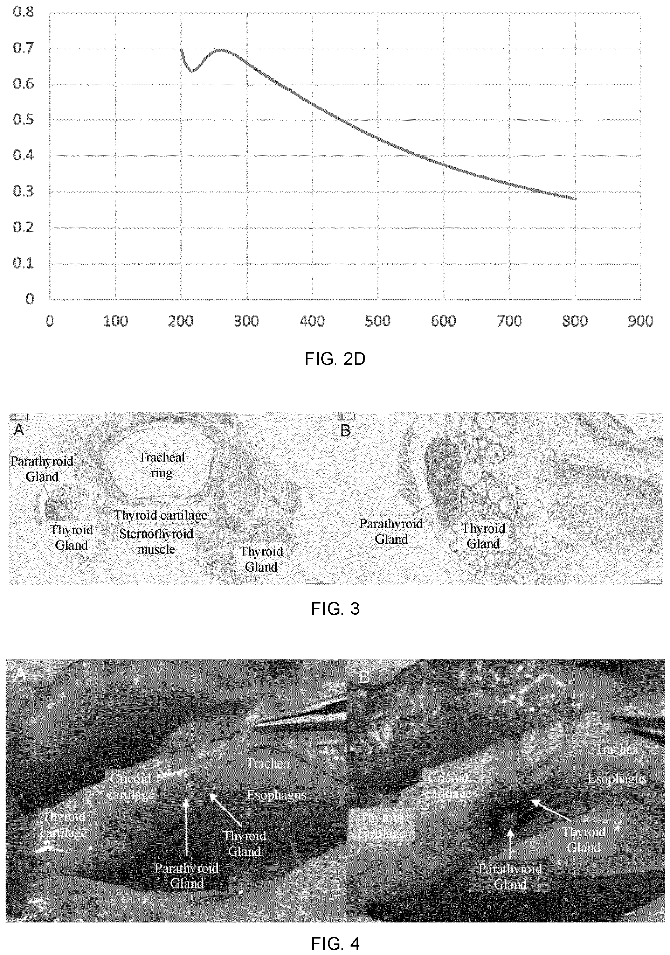

FIG. 2 is an ultraviolet-visible absorption spectrum of the iron oxide nanoparticles; where, FIG. 2A is a UV absorption spectrum of 10 nm iron oxide nanoparticles, FIG. 2B is a UV absorption spectrum of 50 nm iron oxide nanoparticles, FIG. 2C is a UV absorption spectrum of 100 nm iron oxide nanoparticles, and FIG. 2D is a UV absorption spectrum of 150 nm carbon nanoparticles (CNPs, positive reference). The abscissa indicates the wavelength, and the ordinate indicates the absorbance value.
FIG. 3 shows a pathology image of HE-stained parathyroid gland in SD rats; where A shows an image of laryngotrachea passing across the cross-section of parathyroid gland, B shows a locally enlarged view of thyroid gland and parathyroid gland in which the chief cells and oxyphil cells of parathyroid gland in rats can be observed under a microscope.
FIG. 4 shows an anatomical diagram of thyroid gland and parathyroid gland in rats, where A: parts determined as thyroid gland with parathyroid gland by naked eyes, in which parathyroid gland shows transparent; B: negative imaging of parathyroid gland after the injection of iron oxide nanoparticles, in which the thyroid gland is stained to black, and the parathyroid gland remians unstained, which can be obviously determined by naked eyes.

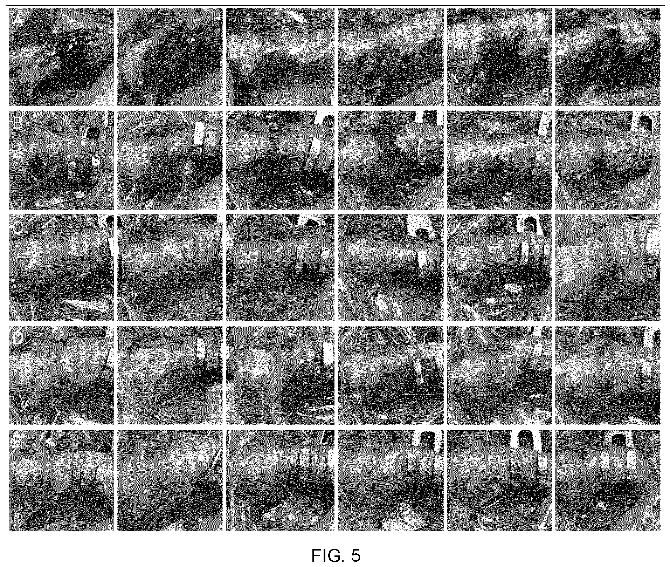

FIG. 5 shows comparation of iron oxide nanoparticles and carbon nanoparticles with different sizes in rats. A, B, C, D, and E represent carbon nanoparticles, 10 nm iron oxide nanoparticles, 50 nm iron oxide nanoparticles, 100 nm iron oxide nanoparticles, and 0.9% normal saline groups, respectively. Side views of 6 rats in each group after an injection of corresponding material into the right thyroid lobe are shown.

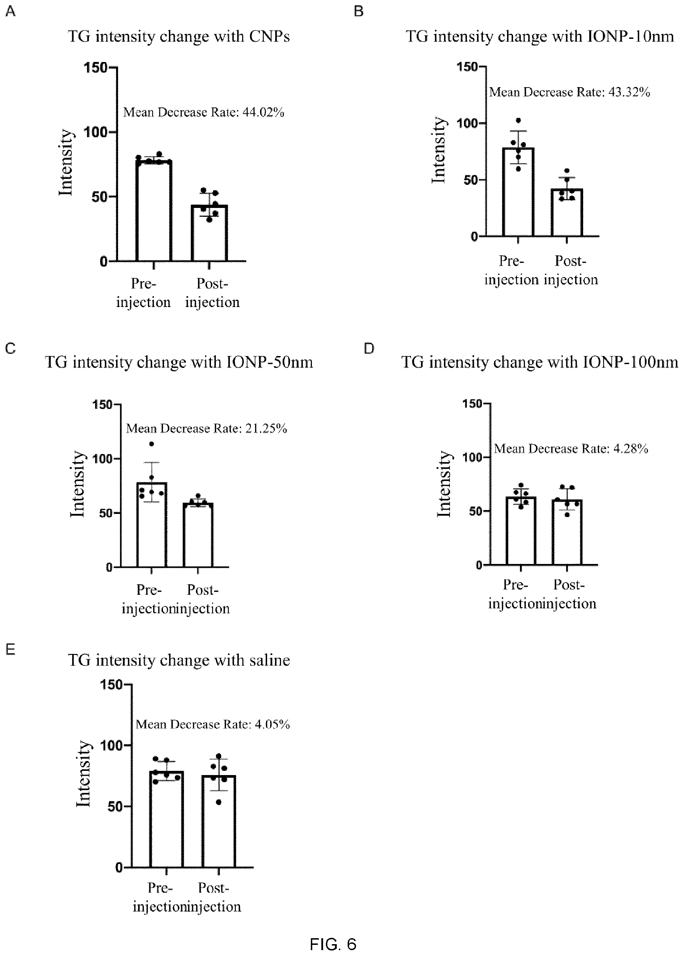

FIG. 6 shows a histogram of the quantitative analysis of the enhancement rate of grayscale before and after the injection of iron oxide nanoparticles with different sizes and carbon nanoparticles in the thyroid gland area. The CNPs group: 44.02%, the 10 nm-IONPs group: 43.32%, the 50 nm-IONPs group: 21.25%, the 100 nm-IONPs group: 4.28%, and the normal saline group: 4.05%.

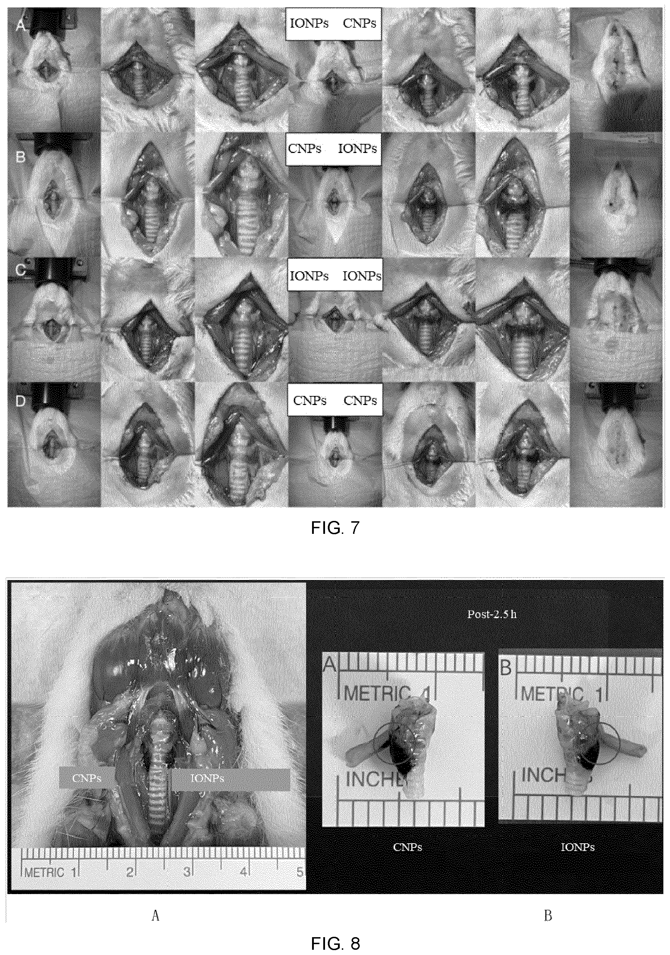

FIG. 7 shows comparative observation experiments of iron oxide nanoparticles and carbon nanoparticles in rats; where A, B, C, and D represent four SD rats, where A: right thyroid lobe and subcutaneous tissue are injected with IONPs, left thyroid lobe and subcutaneous tissue are injected with CNPs; B: right thyroid lobe and subcutaneous tissue are injected with CNPs, left thyroid lobe and subcutaneous tissue are injected with IONPs; C: both left and right thyroid lobes and subcutaneous tissues are injected with IONPs; D: both left and right thyroid lobes and subcutaneous tissues are injected with CNPs.
FIG. 8 shows diagrams of the experiment that injecting iron oxide nanoparticles and carbon nanoparticles into rats and the ex *vivo* diagrams of the esophageal contamination, where: Panel A shows that the left thyroid lobe is injected with CNPs and the right thyroid lobe is injected with IONPs; and it can be seen that the both the left and right thyroid lobes are colored; and the left picture of Panel B shows that 2.5 hours after injection, the CNPs contaminate the esophagus, and the right picture of Panel B shows that 2.5 hours after injection, the IONPs do not contaminate the esophagus, where the adjacent esophagus is not stained to black, as indicated by circles.

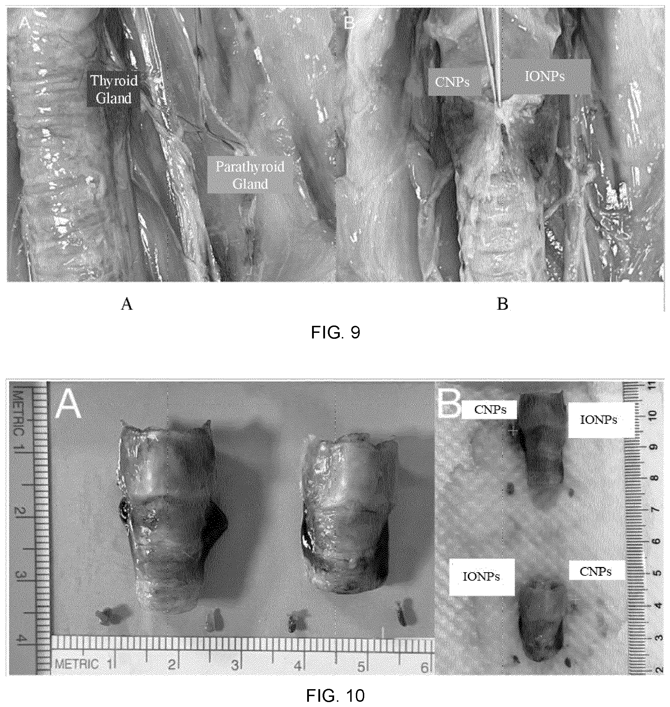

FIG. 9 shows an anatomical diagram of the thyroid gland and parathyroid gland of New Zealand rabbits and an *in vivo* comparison diagram after injection of iron oxide nanoparticles and carbon nanoparticles. Panel A is the anatomical diagram of the thyroid gland and parathyroid gland of New Zealand rabbits, where the thyroid lobes are located on both sides of the trachea, and the parathyroid glands are not located at a constant position, but scattered on the medial side of the anterior cervical muscle. Panel B shows that the right thyroid lobe is injected with CNPs, and the left thyroid lobe is injected with IONPs.
FIG. 10 shows an ex *vivo* comparison diagram of New Zealand rabbits with iron oxide nanoparticles and carbon nanoparticles injected into their thyroid glands. A: The CNPs are injected into one lobe of the thyroid gland and the IONPs are injected into the other lobe of the thyroid gland. The imaging effects are equivalent. B: After 2.5 hours, moistening with normal saline is performed ex *vivo,* and it is observed that the CNPs side exhibits a more obvious leakage than that of the IONPs side, which shows the CNPs are more prone to contaminate the surgical area, as marked with the symbol +.

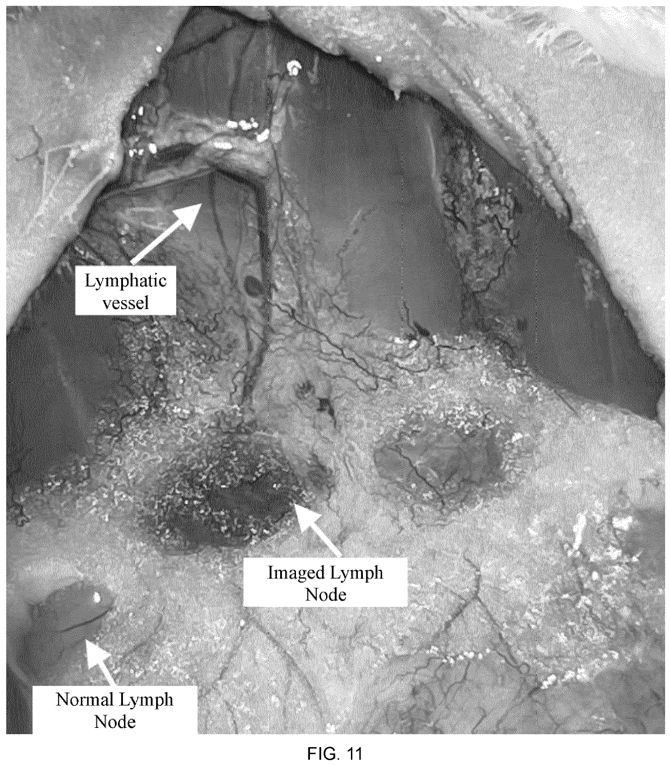

FIG. 11 shows a lymph node imaging diagram by using 10 nm iron oxide nanoparticles.

### DETAILED DESCRIPTION OF THE EMBODIMENTS

Hereinafter the embodiments of the present disclosure are described by the specific examples, and those skilled in the art can easily understand other advantages and effects of the present disclosure from the contents in the description. The present disclosure can also be implemented or applied through other different specific embodiments. Various details in this specification can also be modified or changed in various ways based on different viewpoints and applications without departing from the spirit of the present disclosure. In the specification and the appended claims of the present disclosure, the singular forms "a", "an", and "the" also include plural forms unless clearly dictated otherwise in the context.

When numerical ranges are provided in the embodiments, it should be understood that both the endpoints of each range and any value between the two endpoints are available unless otherwise indicated. Unless otherwise defined, all technical and scientific terms used in the present disclosure have the same meaning as those commonly understood by those skilled in the art. In addition to the specific methods, equipment, and materials used in the embodiments, according to the understanding of those skilled in the art and the disclosure of the present disclosure, any methods, equipment, and materials of the prior art that are similar to or equivalent to the methods, equipment, and materials as described in the embodiments of the present disclosure can be used to realize the present disclosure.

Unless otherwise stated, the experimental methods, detection methods, and preparation methods disclosed in the present disclosure all adopt conventional technologies in the fields of molecular biology, biochemistry, chromatin structure and assay, analytical chemistry, cell culture, recombinant DNA technology, and related fields.

### EXPERIMENTAL METHODS

### 1. Experimental Materials

Carbon nanoparticles: particle size, 150 nm; manufacturer: Chongqing Lummy Pharmaceutical Co., Ltd. (batch number: Z200603; production date: 2020-06-12); black suspension.

Iron oxide nanoparticles (IONPs): particle size, 10 nm, 50 nm, and 100 nm; magnetic nanoparticle solution.

In the following embodiments, the iron oxide nanoparticles used are commercially available. Iron oxide nanoparticles prepared by other methods can also be used, such as those prepared by a thermal decomposition method. The thermal decomposition method includes: heating iron oxide powder and oleic acid in octadecene at high temperature to form IONPs, and then mixing the IONPs in the organic solvent with an aqueous solvent under reflux to transform the IONPs into the aqueous solvent to obtain the iron oxide nanoparticles.

### 2. Experimental Instruments

Nanoparticle size and Zeta-potential analyzer: Zetasizer Nano ZS (Malvern, ZEN3600);

UV-visible absorption spectrophotometer: UV/Vis/NIR Spectrophotometer (PerkinElmer, LAMBDA1050+);

Transmission Electron Microscope (TEM) (JEOL, JEM-2100plus).

### 3. Characterization Indicators of Nanoparticles

Morphological structure, polydispersity index (dispersity or PDI), particle size, Zeta-potential (surface charge (Dynamic Light Scattering, DLS)), ultraviolet-visible absorption spectrum (Ultraviolet-Visible Spectroscopy, UV-Vis).

### Embodiment 1. Characterization of iron oxide nanoparticles and carbon nanoparticles

### 1. Characterization of iron oxide nanoparticles (IONPs)

The particle size and zeta-potential were measured using the Zetasizer Nano ZS (Malvern, ZEN3600) nanoparticle size and potential analyzer. Specifically, a 10 nm-IONPs-containing suspension was diluted with normal saline to an IONP concentration of 50ug/mL, and then the IONP samples were directly placed into a sample cell for measurement. The measurement result is -30.57 ± 0.59mV.

The ultraviolet-visible absorption spectrum was measured by an UV-Vis absorption photometer. An IONP-containing suspension was diluted with normal saline to an IONP concentration of 10 µg/mL, and then the IONP samples were directly placed into the sample cell for measurement. The measurement result is shown in FIG. 2, where, FIG. 2A is the UV-Vis absorption spectrum of 10 nm IONPs with a peak value of 200-220 nm, which is close to the peak value of the UV-Vis absorption spectrum of carbon nanoparticles; FIG. 2B is the UV-Vis absorption spectrum of 50 nm IONPs; and FIG. 2C is the UV-Vis absorption spectrum of 100 nm IONPs.

The morphology structure and the polydispersity index were measured by transmission electron microscopy. A droplet of IONPs with a concentration of 50 µg/mL was dropped onto a copper grid for 30 min, and then the remaining liquid was blotted up. After being thoroughly dried, the copper grid was observed under the electron microscope. The measurement results are: 10 nm, 50 nm, and 100 nm IONPs are all spherical, and the electron microscope size (Fe₃O₄ core diameter) of 10 nm IONPs is about 7 nm.

The measurement methods for carbon nanoparticles are the same as above.

The zeta-potential of carbon nanoparticles is -4.44 ± 0.25. FIG. 2D shows a UV absorption spectrum curve. The particle size is 150 nm; the morphology structure is spherical; the hydrated diameter is 171.50±0.85 nm; and the polydispersity index (PDI) is 0.135.

### Embodiment 2: Pathological HE staining of ex vivo thyroid gland and parathyroid gland of SD rats

One 6-week-old SD rat with weight of about 240g-260g was selected and treated in accordance with the steps 1) to 7) of Embodiment 3. Later, the larynx, trachea, thyroid gland, and parathyroid glands within the range from the superior border of the thyroid gland cartilage to the 5^{th} ring of the trachea were surgically removed, which were then stained with Hematoxylin-Eosin (HE staining for short) and observed under microscope for the pathological results of the rat parathyroid gland.

As shown in FIG. 3, FIG. 3A is an image of the laryngotrachea passing across the cross-section of the parathyroid gland, from which it can be seen that the parathyroid gland clings to the thyroid gland, and is located outside and above the thyroid gland. FIG. 3B is a locally enlarged view of the thyroid gland and the parathyroid gland, where the characteristic chief cells and oxyphil cells of the parathyroid gland can be observed under the microscope (golden standard).

### Embodiment 3. Anatomy and negative imaging of thyroid gland and parathyroid gland in SD rats

6-week-old female and male SD rats with weights of about 240g-260g were selected. The parathyroid glands were negatively imaged according to the blood supply characteristics of the parathyroid gland with selectively traced lymph nodes. The experiment contains 5 groups, with 6 rats in each group, therefore, a total of 5 groups * 6 = 30 rats were investigated.

Experimental groups A, B, and C: correspond to IONPs with three different particle sizes of 10 nm, 50 nm, and 100 nm respectively;
Positive reference: 150 nm CNPs;
Negative reference: PBS solution.

1. Anatomy of normal thyroid gland and parathyroid glands in living SD rats
   1) Anesthetizing 30 rats in the 5 groups by isoflurane inhalation;
   2) Anesthetizing the rats by intraperitoneal administration of chloral hydrate;
   3) Continuously anesthetizing the rats with masks;
   4) Removing hair, disinfecting, and draping;
   5) Making an incision at the middle anterior cervical position, and incising longitudinally;
   6) Isolating the anterior cervical muscles;
   7) Exposing the thyroid gland on both sides;
   8) Taking photographs from the side of the thyroid gland in the living SD rat (marked as M) in the 10 nm IONPs experimental group.
      FIG. 4A shows the anatomy diagram of the thyroid gland and parathyroid gland in rats. Among them, the parts determined as the thyroid gland and parathyroid gland by the naked eyes are indicated by arrows. Usually, the parathyroid gland is transparent, and thus the parathyroid gland is not conspicuous and difficult to be recognized by the naked eyes.
2. Imaging of thyroid gland and parathyroid gland by injection of iron oxide nanoparticles with different sizes

Injection was performed on the thyroid gland of 30 rats in the 5 groups. The negative imaging results are shown in FIG. 5. Each row represents the results of locally injecting one type of material into the thyroid gland of 6 SD rats. Among them, FIG. 5A is the positive control of carbon nanoparticles; FIGS. 5B to 5D are the IONPs experimental groups of 10 nm, 50 nm, and 100 nm, respectively, and FIG. 5E is the blank control of 0.9% normal saline. As seen from FIG. 5, the 10 nm IONPs produce the most enhanced imaging effect.

After injection, the side view of the thyroid gland of the SD rat (marked as M) was photographed. As seen from the result in FIG. 4B, the thyroid gland was stained to black, and the parathyroid gland remained unstained. Therefore, parathyroid gland can be clearly recognized by the naked eyes effectively.

According to FIG. 5, a method for measuring the grayscale decrease before and after clinical injection of CNPs was applied, where the method is in particular as follows: An intraoperative photography was carried out after exposure of the surgical cavity, to compare the change in grayscale of the thyroid gland before and after injection. The grayscale values before and after the injection in circles with the same size that are on the thyroid gland and adjacent to the border of the parathyroid gland were measured by using the grayscale calculation function in ImageJ software. The rate of change in the grayscale of thyroid gland before and after the injection is calculated (by subtracting the grayscale value after the injection from the grayscale value before the injection to obtain a difference, and then dividing the difference by the grayscale value before the injection to obtain the rate of change in the grayscale). The result is shown in FIG. 6. As seen from FIG. 6, 10 nm IONPs (43.32%) have a similar rate of change in the grayscale to CNPs (44.02%), which produces the best enhancement effect. Therefore, 10 nm IONPs with the best imaging effect were used for subsequent experiments.

### Embodiment 4. Anatomy and negative imaging of thyroid gland and parathyroid gland in SD rats

Four 6-week-old rats with weight of about 240-260 g were selected, treated according to the steps 1)-7) of Embodiment 3, and then subjected to injection as follows: A: right thyroid lobe and subcutaneous tissue were injected with IONPs, left thyroid lobe and subcutaneous tissue were injected with CNPs; B: right thyroid lobe and subcutaneous tissue were injected with CNPs, left thyroid lobe and subcutaneous tissue were injected with IONPs; C: both the right and left thyroid lobes and subcutaneous tissues were injected with IONPs; D: both the right and left thyroid lobes and subcutaneous tissues were injected with CNPs.

The imaging results are shown in FIG. 7, where each of FIGs. 7A-D includes 7 pictures from left to right (labeled as A1-A7, B1-B7, C1-C7, D1-D7, respectively). Among them, Images 1, 2, and 3 are blank front views of the rats before the injection (X1, X3, and X5 times); and Images 4, 5, and 6 are the images after the injection of nanomaterials (X1, X3, and X5 times). As seen from the results in Images 1 to 6 of FIGs. 7A-D, the IONPs and the carbon nanoparticles produce similar imaging effects that are not significantly different. In particular, the results in FIG. 7B are similar to those in FIG. 7A. In FIG. 7C, whether the IONPs were injected into the left thyroid lobe or the right thyroid lobe, an efficient imaging enhancement can be observed. In FIG. 7D, whether CNPs are injected into the left thyroid lobe or the right thyroid lobe, an efficient imaging enhancement can be observed.

A7, B7, C7, and D7 show incisions at the middle anterior cervical position, and injections were performed according to the nanoparticle injection method of the respective group to illustrate the comparative degradation of 10 nm IONPs and CNPs in the skin and subcutaneous tissue. That is, A7 shows the subcutaneous injection of the 10 nm IONPs and the CNPs into the left and the right sides of the middle anterior cervical incision, respectively; B7 shows the subcutaneous injection of the CNPs and the 10 nm IONPs into the left and the right sides of the middle anterior cervical incision, respectively; C7 shows the subcutaneous injection of the 10 nm IONPs into both the left and the right sides of the anterior cervical incision; and D7 shows the subcutaneous injection of the CNPs into both the left and the right sides of the anterior cervical incision. As seen from A7 or B7, when different nanoparticles were injected into the left and the right sides of the middle anterior cervical incision of the same rat, respectively, there is no obvious color difference. As seen from C7 or D7, when the same nanoparticles were injected into both the left and the right sides of rats, there is no obvious color difference.

### Embodiment 5. Penetration of nanomaterials into the ex vivo thyroid gland of SD rats after injection

One 6-week-old rat with a weight of about 240g-260g was selected and treated according to the steps 1)-7) of Embodiment 3. Then, the anterior cervical area (including the larynx, trachea, esophagus, thyroid gland, and parathyroid gland) of the SD rat was surgically removed. The left thyroid lobe was injected with CNPs, and the right thyroid lobe was injected with 10 nm IONPs. Photographs were taken from the side, as shown in FIG. 8A. The leakage of nanomaterials was observed. After 2.5 hours, the results are shown in FIG. 8B. On the side to which the CNPs were injected, the esophagus was stained to black (the range shown in the circle), while on the side to which the 10 nm IONPs were injected, the esophagus was not contaminated and showed white (the range shown in the circle), indicating that there is no obvious penetration of the 10 nm IONPs into the parathyroid gland area, and the imaging effect is better.

Based on the above, it can be seen from FIGs. 4 to 8 that in the imaging of ex *vivo* or *in vivo* thyroid gland, the 10 nm IONPs and the CNPs have similar enhancement effects, but the 10 nm IONPs are less likely to leak and thus have significantly better performance than CNPs.

### Embodiment 6. Anatomy and negative imaging of thyroid gland and parathyroid gland in New Zealand rabbits

The parathyroid glands were negatively imaged according to the blood supply characteristics of the parathyroid gland with selectively traced lymph nodes.

Experimental group: Injected with 10 nm IONPs:
1. Anatomy of thyroid gland and parathyroid gland in New Zealand rabbits
   1) Weighing and selecting New Zealand rabbits with weights of about 3 kg - 3.5 kg;
   2) Anesthetizing the rabbits by intraperitoneal administration of chloral hydrate;
   3) Making an incision at the middle anterior cervical position, and incising longitudinally;
   4) Isolating the anterior cervical muscles;
   5) Exposing the thyroid gland and the parathyroid glands on both sides;
   6) Taking photographs from the front side of the thyroid gland in the living rabbit.
      FIG. 9A shows the anatomy diagram of the thyroid gland and parathyroid gland of New Zealand rabbits. It can be seen that thyroid gland is located on both sides of the trachea, and the position of the parathyroid glands is not constant and is scattered on the inner surface of the anterior cervical muscle.
2. Imaging effect of thyroid gland and parathyroid gland in living New Zealand rabbits after injection of nanomaterials
   7) Injecting the 10 nm IONPs locally into the left thyroid lobe of the above-mentioned New Zealand rabbits, and injecting the CNPs into the right thyroid lobe (performing comparison between the IONPs and CNPs in the same rabbit left-right compartments);
   8) Then, photographing the thyroid gland of the living New Zealand rabbits, both lobes were imaged. The results are shown in FIG. 9B. In the New Zealand rabbit animal models, both the 10 nm IONPs and the CNPs showed comparable image enhancement effect.

### Embodiment 7. Penetration of ex vivo thyroid gland of New Zealand rabbit after injection of nanomaterials

Two New Zealand rabbits with weights of about 3 kg - 3.5 kg were selected and treated according to the steps 1)-5) of Embodiment 6. Then, the thyroid gland, parathyroid gland, larynx, and trachea of the New Zealand rabbits were surgically removed. The thyroid gland on the left and the right sides of the New Zealand rabbits was injected with the CNPs and the 10 nm IONPs, respectively, and the results are shown in FIG. 10A. It can be seen that both the 10 nm IONPs and the CNPs showed comparable image enhancement on the thyroid lobes. After 2-3 hours, as shown in FIG. 10B, on the side to which the CNPs were injected, an obvious leakage occurred, which contaminated the surgical area, and was stained to black, which is marked with the symbol "+" in the drawing, and on the side to which the IONPs were injected, the border was clear, and no contamination or leakage occurred (where the left tissue in FIG. 10A corresponds to the upper tissue in FIG. 10B, and the right tissue in FIG. 10A corresponds to the lower tissue in FIG. 10B).

It can be seen from the imaging results in FIGs.9 and 10 that the 10 nm IONPs of the present disclosure are equivalent to the CNPs in the negative imaging of the thyroid gland of New Zealand rabbits, but a surrounding contamination is more prone to occur in the CNPs group.

### Embodiment 8. Positive imaging of lONPs in rat submandibular lymph nodes

The principle of lymph node imaging: after local injection (lower lip) of 10 nm IONPs, the IONPs were transported to the submandibular lymph nodes via the anterior cervical lymphatic vessels. The submandibular lymph nodes in rats were relatively superficial, and thus black-stained lymph nodes can be observed after incising the skin.
1) 6-week-old SD rats with weight of about 240 g-260 g were selected after weighing;
3) Anesthetizing the rats by isoflurane inhalation;
4) Anesthetize the rats by intraperitoneal injection of 10% chloral hydrate (calculated at 300 ul/g);
5) Continuously anesthetizing the rats with masks;
6) Removing hair, disinfecting, and draping;
7) Injecting 60 ul of 10 nm IONPs with a concentration of 20 mg/ml into three points in the lower lip of the rats, with 20 ul per point;
8) Making an incision at the middle anterior cervical position, and incising longitudinally to a length of about 3-4 cm;
9) Observing the staining of lymph nodes in the submandibular area. As shown in FIG. 11, the lymphatic vessels communicating the lower lip and the lymph nodes can be observed, which are elongated, dark brown, and accompanied with blood vessels. The lymph nodes communicated with the corresponding lymphatic vessels exhibit a darker color than normal lymph nodes, and are dark brown. Normal lymph nodes in the control group without injection are white. It can be seen that the IONPs can be used to image the lymph nodes, and can be used as tracers for lymph nodes or delivery carriers for delivering related drugs to lymph nodes.

The foregoing embodiments only illustrate the principles and effects of the present disclosure, but are not intended to limit the present disclosure. Any person skilled in the art can modify or change the above embodiments without departing from the spirit and scope of the present disclosure. Therefore, all equivalent modifications or changes made by persons skilled in the art without departing from the spirit and technical concepts disclosed in the present disclosure should still be encompassed by the appended claims of the present disclosure.

## Claims

1. A use of iron oxide nanoparticles IONPs in the preparation of a contrast agent, wherein the IONPs have a nanoparticle structure, with a particle size ranging from 1 nm to 1000 nm, a zeta-potential ranging from -50 mV to 50 mV, and a peak value of ultraviolet-visible absorption spectrum ranging from 100 nm to 500 nm.

2. The use according to claim 1, wherein the IONPs have a particle size ranging from 1 nm to 200 nm, a zeta-potential ranging from -35 mV to 20 mV, and a peak value of ultraviolet-visible absorption spectrum ranging from 180 nm to 300 nm.

3. The use according to claim 1, wherein the IONPs have one or more particle sizes ranging from 1 nm to 100 nm, a zeta-potential ranging from -35 mV to 0 mV, and a peak value of ultraviolet-visible absorption spectrum ranging from 200 nm to 220 nm.

4. The use according to any one of claims 1 to 3, wherein the contrast agent is a negative contrast agent for parathyroid glands and/or a positive contrast agent for lymph nodes.

5. The use according to any one of claims 1 to 3, wherein the contrast agent is used for tissue imaging of thyroid glands, parathyroid glands, and/or lymph nodes or used in intraoperative navigation.

6. A contrast agent, comprising the iron oxide nanoparticles IONPs as described in the use of any one of claims 1 to 3.

7. A diagnostic tracer, comprising the iron oxide nanoparticles IONPs as described in the use of any one of claims 1 to 3.

8. A pharmaceutical composition, comprising the iron oxide nanoparticles IONPs as described in the use of any one of claims 1 to 3 and a pharmaceutically acceptable carrier or medium.

9. A use of the pharmaceutical composition of claim 8 in the preparation of a product for diagnosis and/or treatment of thyroid gland and/or lymph nodes.

10. A method of screening an effective therapeutic drug to treat thyroid gland and/or lymph nodes, comprising:
constructing IONPs and drug molecules into a single carrier to form a drug, and
evaluating the efficacy of the drug for thyroid gland and/or lymph nodes by the effectiveness of IONPs on the thyroid gland positive contrast imaging, the parathyroid gland negative contrast imaging, and/or the lymph node positive contrast imaging.
